# EUROPÄISCHE PATENTSCHRIFT

(11) **EP 2 453 984 B1**
(45) Veröffentlichungstag und Bekanntmachung des Hinweises auf die Patenterteilung: **03.01.2018**
(21) Anmeldenummer: 10730121.0
(22) Anmeldetag: 18.06.2010
(51) Int. Cl.: A61N 5/10

(54) **BESTRAHLUNG BZW. BESTRAHLUNGSPLANUNG FÜR EIN RESCANNING-VERFAHREN MIT EINEM PARTIKELSTRAHL**
IRRADIATION OR IRRADIATION PLANNING SYSTEM FOR A RESCANNING METHOD USING A PARTICLE BEAM
IRRADIATION OU PLANIFICATION D'IRRADIATION POUR UN PROCÉDÉ À BALAYAGE RÉPÉTÉ AVEC UN FAISCEAU DE PARTICULES

(30) Priorität: 15.07.2009 DE 102009033297
(43) Veröffentlichungstag der Anmeldung: 23.05.2012
(73) Patentinhaber: GSI Helmholtzzentrum für Schwerionenforschung GmbH, 64291 Darmstadt (DE)
(72) Erfinder: BERT, Christoph, 63741 Aschaffenburg (DE); RIETZEL, Eike, 64331 Weiterstadt (DE)
(74) Vertreter: Mergel, Volker
(86) Internationale Anmeldenummer: PCT/EP2010/058636
(87) Internationale Veröffentlichungsnummer: WO 2011/006733

(56) Entgegenhaltungen:
- DE-A1-102008 027 485
- Silvian Zenklusen: "Preliminary investigation for Developping repainted beam scanning on the PSI Gantry 2" 24. Mai 2008 (2008-05-24), XP002601203 Gefunden im Internet: URL:http://ptcog.web.psi.ch/ptcog47_talks. html [gefunden am 2010-09-15]
- NAMI SAITO ET AL: "Speed and accuracy of a beam tracking system for treatment of moving targets with scanned ion beams; Speed and accuracy of a beam tracking system" PHYSICS IN MEDICINE AND BIOLOGY, TAYLOR AND FRANCIS LTD. LONDON, GB, Bd. 54, Nr. 16, 21. August 2009 (2009-08-21), Seiten 4849-4862, XP020158936 ISSN: 0031-9155

## Beschreibung

Die Erfindung ist in den Ansprüchen definiert. Die Erfindung betrifft ein Verfahren zur Bestrahlungsplanung eines Zielvolumens, ein Verfahren zur Bestrahlung eines Zielbereichs in einem Zielvolumen, eine Bestrahlungsplanungsvorrichtung, eine Steuerungsvorrichtung für eine Bestrahlungsanlage sowie eine Bestrahlungsanlage mit einer derartigen Steuerungsvorrichtung, welche ein Rescanning-Bestrahlungsverfahren implementieren oder ermöglichen. Ein derartiges Bestrahlungsverfahren wird insbesondere zur Bestrahlung von bewegten Zielvolumina eingesetzt zur Kompensation der durch die Bewegung des Zielvolumens hervorgerufenen Bestrahlungsungenauigkeiten.
Die Partikeltherapie ist ein etabliertes Verfahren zur Behandlung von Gewebe, insbesondere von Tumorerkrankungen. Bestrahlungsverfahren, wie sie in der Partikeltherapie eingesetzt werden, finden jedoch auch in nicht-therapeutischen Gebieten Anwendung. Hierzu gehören beispielsweise Forschungsarbeiten, etwa zur Produktentwicklung, im Rahmen der Partikeltherapie, die an nicht-lebenden Phantomen oder Körpern durchgeführt werden, Bestrahlungen von Materialien, etc.
Hierbei werden geladene Partikel wie z.B. Protonen oder Kohlenstoffionen oder andere Ionen auf hohe Energien beschleunigt, zu einem Partikelstrahl geformt und über ein Hochenergiestrahltransportsystem zu einem oder mehreren Bestrahlungsräumen geführt. In dem Bestrahlungsraum wird das zu bestrahlende Zielvolumen mit dem Partikelstrahl bestrahlt.
Es kann dabei vorkommen, dass sich das zu bestrahlende Zielvolumen bewegt. Beispielsweise kann bei der Bestrahlung eines Patienten eine Bewegung des zu bestrahlenden Tumors durch eine Atembewegung verursacht werden. Eine derartige Bewegung kann auch anhand von als Phantomen bezeichneten Modellobjekten für Forschungszwecke nachgebildet werden.

Insbesondere bei Bestrahlungsverfahren, bei denen eine Vielzahl von Bestrahlungsdosen sukzessive an unterschiedlichen Orten im Zielvolumen deponiert werden soll, also bei einem gescannten Partikelstrahl, ist es schwer, eine gewünschte homogene Dosisverteilung im Zielvolumen zu erreichen, wenn sich das Zielvolumen während des Fortgangs der Bestrahlung bewegt.

Bei einem gescannten Partikelstrahl ist es daher möglich, die zu applizierende Dosis auf mehrere Durchgänge zu verteilen. Dieses Verfahren ist auch unter dem Namen "Rescanning" bekannt. Dies bedeutet, dass ein Zielbereich mehrfach abgefahren wird und dass dort die Gesamtdosis sukzessive durch mehrere, wiederholt applizierte Einzeldosen während der Rescan-Durchgänge aufgebaut wird. Dies hat den Vorteil, dass sich Fehler bei der Dosisdeposition, die bei einem einzigen Durchgang zu einer vollständig fehlerhaft deponierten Dosis führen würde, durch die mehreren Rasterscan-Durchgänge bis zu einem gewissen Grad ausmitteln. Lageunsicherheiten bzgl. des Zielvolumens, Bewegungen des Zielvolumens etc. können so zumindest teilweise kompensiert werden.

Der online veröffentliche Vortrag von Silvan Zenklusen et al. "Preliminary investigation for developing repainted beam scanning on the psi gantry 2", erhältlich unter http://ptcog.web.psi.ch/ptcog47_talks.html, schlägt zur Verbesserung vor, bei jedem Zielpunkt eines Bereichs, der mehrfach in mehreren Rescan-Durchgängen gescannt wird, bei jedem Rescan-Durchgang stets eine Dosis zu applizieren, die kleiner ist als eine Obergrenze, ein "Upper-Dose-Limit". Ein Zielpunkt wird in konsekutiven Rescan-Durchgängen so lange bestrahlt, bis seine Soll-Dosis erreicht wurde. In den anschließenden Rescan-Durchgängen wird dieser Zielpunkt von einer weiteren Bestrahlung ausgenommen, d.h. dieser Zielpunkt wird nicht mehr angefahren.

Es ist die Aufgabe der Erfindung, ein Verfahren zur Bestrahlungsplanung bzw. ein Verfahren zur Bestrahlung anzugeben, das beim Rescannen eine schnelle und vorteilhafte Ansteuerung der Bestrahlungsanlage erlaubt. Weiterhin ist es die Aufgabe der Erfindung eine entsprechende Bestrahlungsplanungsvorrichtung, Steuerungsvorrichtung für eine Bestrahlungsanlage und Bestrahlungsanlage bereitzustellen.

Die Erfindung wird gelöst durch die Gegenstände der unabhängigen Ansprüche. Bevorzugte Ausgestaltungen der Erfindung sind in den abhängigen Ansprüchen angegeben und werden im Folgenden näher erläutert. Die vorangehende und die folgende Beschreibung der einzelnen Merkmale bezieht sich sowohl auf die Vorrichtungskategorie als auch auf die Verfahrenskategorie, ohne dass dies im Einzelnen in jedem Fall explizit erwähnt ist; die dabei offenbarten Einzelmerkmale können auch in anderen als den gezeigten Kombinationen erfindungswesentlich sein.

Das erfindungsgemäße Verfahren zur Bestrahlungsplanung eines Zielvolumens umfasst folgende Schritte:
- Festlegen eines Zielbereichs mit einzeln anfahrbaren Zielpunkten,
- Festlegen einer Anzahl von Rescan-Durchgängen, in denen der Zielbereich mehrfach abgescannt wird, derart, dass die Zielpunkte des Zielbereichs während der Rescan-Durchgänge unterschiedlich oft angefahren werden, wodurch zumindest ein Teil der Zielpunkte nicht bei jedem Rescan-Durchgang angefahren wird,
wobei das Anfahren der Zielpunkte derart auf die Rescan-Durchgänge aufgeteilt wird, dass bei zumindest einem Zielpunkt, der nicht in jedem Rescan-Durchgang angefahren wird, vor dem letzten Rescan-Durchgang, bei dem dieser Zielpunkt angefahren wird, zumindest ein weiterer Rescan-Durchgang liegt, bei dem dieser Zielpunkt nicht angefahren wird.

Das zu bestrahlende Zielvolumen ist dabei üblicherweise in eine Mehrzahl von Zielbereichen aufgeteilt. Während einer Bestrahlungssitzung werden die Zielbereiche im Rescanning-Verfahren bestrahlt, d.h. jeweils mit mehreren Rescan-Durchgängen. Die Zielbereiche werden dabei üblicherweise sukzessive abgetastet, d.h., sobald ein Zielbereich im Rescanning-Verfahren abgetastet wurde, wird der nächste Zielbereich wiederum im Rescanning Verfahren abgetastet, usw.

Insbesondere kann die Dosisverteilung, die für den Zielbereich festgelegt wird, inhomogen sein. Selbst wenn die Gesamtdosis, die für das Zielvolumen appliziert werden soll, eine homogene Dosisverteilung aufweist, kann es notwendig sein, dass die zu applizierende Dosisverteilung für einen Zielbereich inhomogen ist. Dies rührt daher, dass Teile des Zielbereichs bereits während der Bestrahlung anderer Zielbereiche mit einer Vor-Dosis belegt wurden. Die Dosisverteilung kann dabei ein Maß für die zu applizierende Partikelzahl darstellen. Die Dosisverteilung kann unter Berücksichtigung von Planungsvorgaben hinsichtlich Zielvolumen, der im Zielvolumen zu deponierenden Dosis und/oder der effektiven Wirkung der im Gewebe deponierten Dosis - beispielsweise charakterisierbar durch die Angabe der relativen biologische Wirksamkeit (RBW bzw. RBE) - in einer Planungsphase ermittelt werden.

Bei dem erfindungsgemäßen Verfahren wird die Dosisverteilung für den Zielbereich dadurch appliziert, dass die Zielpunkte des Zielbereichs während der Rescan-Durchgänge unterschiedlich oft angefahren werden. Unter "Anfahren eines Zielpunktes" wird dabei verstanden, dass in dem Zielpunkt eine Einzeldosis appliziert wird bzw. dass dies geplant ist. Insbesondere eine inhomogene Dosisverteilung kann auf diese Weise einfach appliziert werden. So kann beispielsweise ein Zielpunkt, für den eine höhere Gesamtdosis vorgesehen ist, während der Rescan-Durchgänge insgesamt öfter angefahren werden als ein Zielpunkt, für den eine geringere Gesamtdosis vorgesehen ist.

Dadurch, dass die Zielpunkte des Zielbereichs unterschiedlich oft angefahren werden, ergibt sich, dass zumindest ein Teil der Zielpunkte bei bestimmten Rescan-Durchgängen nicht angefahren wird. Wenn beispielsweise insgesamt 10 Rescan-Durchgänge vorgesehen sind, ein Zielpunkt jedoch nur siebenmal insgesamt angefahren werden soll, existieren 3 Rescan-Durchgänge, bei denen der Zielpunkt nicht angefahren wird, d.h. übersprungen wird.

Der Erfindung liegt die Idee zu Grunde, dass es vorteilhaft ist, wenn das Anfahren der Zielpunkte derart auf die Rescan-Durchgänge aufgeteilt wird, dass bei zumindest einem Zielpunkt, der nicht in jedem Rescan-Durchgang angefahren wird, vor dem letzten Rescan-Durchgang, bei dem dieser Zielpunkt angefahren wird, zumindest ein weiterer Rescan-Durchgang liegt, bei dem dieser Zielpunkt nicht angefahren wird. Dies kann beispielsweise erreicht werden, indem dieser Zielpunkt nicht bei dem ersten Rescan-Durchgang angefahren wird. Dies kann aber auch erreicht werden, indem bei einem Zielpunkt, der bei zumindest zwei Rescan-Durchgängen angefahren wird, zwischen diesen zwei Rescan-Durchgängen zumindest ein weiterer Rescan-Durchgang liegt, bei dem der Zielpunkt nicht angefahren wird.

Es ist zwar vorteilhaft, wenn beim Rescannen die Zielpunkte unterschiedlich oft angefahren werden. Dadurch ist es nämlich möglich, eine inhomogene Dosisapplikation zu gewährleisten und gleichzeitig darauf zu achten, dass keine Einzeldosis, die an einem Zielpunkt appliziert wird, derart klein wird, dass mit den Messinstrumenten eine sichere Überwachung der Applikation der Einzeldosis nicht mehr gewährleistet werden kann.

Es wurde aber auch erkannt, dass es problematisch ist, wenn bei wiederholten Rescan-Durchgängen die Zielpunkte solange angefahren werden, bis die Gesamtdosis pro Zielpunkt appliziert worden ist und diese Zielpunkte in folgenden Rescan-Durchgängen nicht mehr angefahren werden. Durch dieses "starre" Schema dünnt sich die Menge der Zielpunkte aus, die in einem Rescan-Durchgang angefahren werden. Dies ist aber unvorteilhaft für eine Bestrahlungsanlage. Wenn in einem späteren Rescan-Durchgang, bei dem die Zielpunkte ausgedünnt sind, der Strahl deshalb zu oft unterbrochen werden muss, beispielsweise um von einer verbleibenden Insel von Zielpunkten zur nächsten verbleibenden Insel zu gelangen, erhöht dies die Bestrahlungszeit.

Dieses starre Schema ist durch das vorliegende Planungsverfahren aufgebrochen. Hierdurch ist es möglich, die Bestrahlung eines Zielpunktes auf einen späteren Zeitpunkt, d.h. auf einen späteren Rescan-Durchgang zu verschieben. Dies ermöglicht es, die beschriebenen negativen Effekte zu verringern oder zu vermeiden.

Beispielsweise kann ein Zielpunkt, der erst bei einem späteren Rescan-Durchgang angefahren wird, dazu verwendet werden, die verbleibenden Inseln des späteren Rescan-Durchgangs zu verbinden. Hierdurch kann der Strahlpfad, der während des späteren Rescan-Durchgangs abgefahren wird, flexibel optimiert werden. Auch wenn üblicherweise mehrere Zielpunkte verwendet werden, um die verbleibenden Inseln eines späteren Rescan-Durchgang zu verbinden, kann - je nach Konstellation der Geometrie des Zielbereichs und der zu applizierenden Dosisverteilung - bereits ein einzelner Zielpunkt, der erst während eines späteren Rescan-Durchgangs angefahren wird, zu einer verbesserten Ausführung der Rescan-Durchgänge führen.

Die während der Rescan-Durchgänge anzufahrenden Zielpunkte können aber auch derart aufgeteilt werden, dass nun möglichst gleichmäßig viele Zielpunkte pro Rescan-Durchgang angefahren werden, d.h. dass die Zahl der Zielpunkte, die bei jedem Durchgang angefahren werden, im Wesentlichen gleich ist. Diese im Wesentlichen gleichmäßige Verteilung bewirkt, dass ein "Ausdünnen" nicht oder in nur sehr geringem Maße auftritt. Dies kann beispielsweise dadurch erreicht werden, dass bei jedem Rescan-Durchgang ein oder mehrere Zielpunkte existieren, die nicht angefahren werden. Die Zielpunkte und die Anzahl der Anfahrten pro Zielpunkt können statistisch über die Rescan-Durchgänge verteilt werden.

Eine Möglichkeit, das Aufteilen der Zielpunkte auf die Rescan-Durchgänge vorzunehmen, ist es das Aufteilen im Hinblick auf den Scan-Pfad vorzunehmen, und zwar derart, dass ein Scan-Pfad, mit dem in einem der Rescan-Durchgänge die anzufahrenden Zielpunkte angefahren werden, ein vordefiniertes Kriterium erfüllt. Beispielsweise kann nach einem Aufteilen der Zielpunkte geprüft werden, ob bei jedem Rescan-Durchgang der daraus resultierende Scan-Pfad die vordefinierten Kriterien erfüllt. Falls dies nicht der Fall ist, kann das Aufteilen der Zielpunkte verändert werden. Das Aufteilen kann auch von vornherein so gestaltet werden, dass bei dem durchzuführenden Algorithmus bestimmte Kombinationen von Zielpunkten und Rescan-Durchgängen nicht erlaubt oder bestraft werden.

Das vordefinierte Kriterium kann beispielsweise sein, dass in einem Scan-Pfad ein Abstand zwischen zwei nacheinander anzufahrenden Zielpunkten unterhalb einer Schwelle liegt. Beispielsweise kann festgelegt werden, dass der maximale Abstand zwischen zwei Zielpunkten geringer als 20 mm, insbesondere geringer als 10 mm oder 5 mm ist. In diesem Fall kann nämlich das Scannen erfolgen, ohne dass eine Abschaltung des Strahls zwischen den zwei Zielpunkten zwingend notwendig ist. Andere mögliche Kriterien können sein: Die Anzahl der notwendigen Unterbrechungen des Strahls beim Abfahren des Scan-Pfads, welche es zu minimieren gilt oder welche unterhalb eines Schwellwertes liegen sollen.

In einer vorteilhaften Ausführungsvariante wird das Planungsverfahren dahin gehend ausgestaltet, dass bei einem Zielpunkt die zu applizierende Gesamtdosis ein ganzzahliges Vielfaches der bei diesem Zielpunkt zu applizierenden Einzeldosis ist, die immer dann appliziert wird, wenn der Zielpunkt in einem Rescan-Durchgang angefahren wird. Diese Einschränkung hat den Vorteil, dass in jedem Zielpunkt stets die gesamte Einzeldosis appliziert werden kann. Die Applikation von Bruchteilen der Einzeldosis, die mitunter problematisch ist, da sie eventuell nicht mit der gebotenen Genauigkeit überwacht werden kann, wird dadurch vermieden.

Auch diese Ausführungsvariante kann unabhängig von dem offenbarten Verfahren implementiert werden. Dies bedeutet, dass beim Rescanning bzw. bei einer Bestrahlungsplanung für ein Rescanning-Verfahren pro Zielpunkt lediglich Verhältnisse zwischen Gesamtdosis und Einzeldosis zugelassen werden, die ganzzahlig sind. Auf diese Weise kann sichergestellt werden, dass pro Zielpunkt stets die gesamte Einzeldosis appliziert wird. Implementieren lässt sich eine derartige Ausgestaltung z.B. durch Vorgabe von Randbedingungen, welche die Freiheiten bei der Bestimmung der Bestrahlungsparameter einschränken. Beispielsweise kann auch die räumliche Lage der Zielpunkte so gewählt werden, dass sich diese Vorgabe erfüllen lässt. Die Einzeldosis, die dem ganzzahligen Verhältnis zu Grunde liegt, kann für den gesamten Bestrahlungsplan gleich gewählt werden, oder auch für unterschiedliche Abschnitte des Bestrahlungsplans - wie für unterschiedliche Zielbereiche - unterschiedlich.

Es ist vorteilhaft, die Einzeldosis derart klein zu wählen, dass sie gerade noch oberhalb eines Schwellwertes liegt. Hierdurch lässt sich nämlich sicherstellen, dass die Dosisapplikation genau überwacht werden kann. Wenn eine Einzeldosis nämlich zu klein würde, gelingt es mitunter nicht mehr, die Einzeldosis mit hinreichender Genauigkeit durch die Messinstrumente zu überwachen, was letztlich den Behandlungserfolg gefährden kann.

Auch unabhängig von den offenbarten Verfahren kann es vorteilhaft sein, die in einem Zielpunkt zu applizierende Einzeldosis, also die Dosis, die bei einem Anfahren des Zielpunktes appliziert wird, insbesondere beim Rescanning so zu wählen, dass die Einzeldosis stets oberhalb eines vorgegebenen Schwellwertes liegt. Auf diese Weise kann sichergestellt werden, dass die Einzeldosis noch von Messinstrumenten, mit denen die Dosisapplikation überwacht wird, mit der gebotenen Genauigkeit überwacht werden kann. Die Einzeldosis kann auf die Wahl des Messbereichs der Messinstrumente und auf die extrahierte Intensität aus dem Beschleuniger abgestimmt sein; insgesamt werden die Möglichkeiten der verwendeten Hardware explizit berücksichtigt. Falls im Laufe der Bestrahlung die Hardware in einem anderen Betriebsmodus betrieben wird, z.B. mit geänderter extrahierter Intensität und/oder mit einer anderen Wahl des Messbereichs der Detektoren, kann der vorgegebene Schwellwert angepasst werden.

Dennoch soll die Einzeldosis innerhalb dieser Vorgabe möglichst klein gewählt werden, da dann nämlich die Anzahl der Rescan-Durchgänge erhöht wird und somit die positiven Effekte des Rescanning deutlicher hervortreten. Zudem kann die Vorgabe "Gesamtdosis ist ein ganzzahliges Vielfaches der Einzeldosis" einfacher erfüllt werden. Beispielsweise kann die Einzeldosis stets unterhalb des Zehnfachen bzw. des Fünffachen des Schwellwertes liegen, insbesondere unterhalb des Zweifachen des Schwellwertes. Bestrahlungsplanungsvorrichtungen bzw. Steuerungsvorrichtungen für Bestrahlungsanlagen können entsprechend ausgebildet werden.

In einer vorteilhaften Ausführung wird bei den Zielpunkten des Zielbereichs stets jeweils eine gleich große Einzeldosis appliziert. Diese Ausführungsvariante ist besonders geeignet, auf einer Bestrahlungsanlage installiert zu werden, weil eine Bestrahlungsanlage auf die zu applizierende gleichgroße Einzeldosis hin ausgelegt und optimiert werden kann. Beispielsweise können die Messbereiche der Vorrichtungen, mit denen die Applikation der Einzeldosis überwacht wird, auf die Größe der Einzeldosis abgestimmt werden. Des Weiteren kann es beim Rescanning und insbesondere beim volumetrischen Rescanning auftreten, dass die Bestrahlung ohne Umschalten von Isoenergieschichten und/oder ohne Umschalten der Intensität, mit der der Partikelstrahl von der Beschleunigereinheit extrahiert wird, und/oder ohne Umschalten der Messbereiche der Messvorrichtungen wie z.B. des Ionisationskammersystems erfolgt, was in einer gleich großen Einzeldosis resultiert.

Es ist dabei nicht notwendig, den Zielbereich komplett mit allen Rescan-Durchgängen abzuscannen, bevor zu einem weiteren Zielbereich des Zielvolumens übergegangen wird, der dann seinerseits mit verschiedenen Rescan-Durchgängen bestrahlt wird. Bereits während einiger Rescan-Durchgänge, mit denen der eine Zielbereich bestrahlt wird, können Zielpunkte eines anderen Zielbereichs angefahren werden. Auch durch dieses "Verweben" der Rescan-Durchgänge für den einen Zielbereich mit den Rescan-Durchgängen des anderen Zielbereichs kann ein vorteilhaftes Anfahren der Zielpunkte erreicht werden. Wenn bei einem Rescan-Durchgang beispielsweise nur mehr wenige Zielpunkte eines Zielbereichs angefahren werden müssten, ist es denkbar, diese Zielpunkte auch in einem anderen Rescan-Durchgang des anderen Zielbereichs unterzubringen. D.h. mitunter ist es vorteilhaft, noch einen Teil der Zielpunkte eines Zielbereichs erst dann zu abzufahren, wenn bereits die Bestrahlung eines anderer Zielbereichs begonnen wurde. Auch dieses Verfahren kann unabhängig von den offenbarten Verfahren implementiert werden. Bestrahlungsplanungsvorrichtungen bzw. Steuerungsvorrichtungen für Bestrahlungsanlagen können entsprechend ausgebildet werden.

Das erfindungsgemäße Verfahren zur Bestrahlung eines Zielbereichs in einem Zielvolumen ist dahingehend ausgestaltet, dass der Zielbereich einzeln anzufahrende Zielpunkte umfasst und dass im Zielbereich insbesondere eine inhomogene Dosisverteilung appliziert wird. Der Zielbereich wird mit einer Vielzahl von Rescan-Durchgängen bestrahlt, d.h. der Zielbereich wird insgesamt also mehrfach abgescannt. Die Zielpunkte des Zielbereichs selbst werden während der Rescan-Durchgänge unterschiedlich oft angefahren, so dass zumindest ein Teil der Zielpunkte nicht bei jedem Rescan-Durchgang angefahren wird. Das Verfahren ist dahingehend ausgestaltet, dass bei zumindest einem Zielpunkt, der nicht in jedem Rescan-Durchgang angefahren wird, vor dem letzten Rescan-Durchgang, bei dem dieser Zielpunkt angefahren wird, zumindest ein weiterer Rescan-Durchgang ausgeführt wird, bei dem dieser Zielpunkt nicht angefahren wird.

Die erfindungsgemäße Bestrahlungsplanungsvorrichtung weist eine Rechnereinheit auf, welche zur Durchführung eines der offenbarten Verfahren zur Bestrahlungsplanung ausgebildet ist. Dies kann beispielsweise mithilfe eines geeigneten Computerprogramms geschehen.

Die erfindungsgemäße Steuerungsvorrichtung für eine Bestrahlungsanlage weist einen Steuerungsrechner auf, der eine Bestrahlungsanlage während eine Bestrahlung derart steuert, dass eines der offenbarten Verfahren zur Bestrahlung eines Zielbereichs in einem Zielvolumen ausgeführt wird. Auch dies kann mit einem geeigneten Computerprogramm geschehen. Die erfindungsgemäße Bestrahlungsanlage weist eine derartige Steuerungsvorrichtung auf.

Ausgestaltungen und Vorteile, wie sie für das Verfahren zur Bestrahlungsplanung aufgeführt und erläutert worden sind, gelten ebenso für das Verfahren zur Bestrahlung eines Zielbereichs in einem Zielvolumen, und in entsprechender Weise für die Bestrahlungsplanungsvorrichtung und für die Steuerungsvorrichtung für eine Bestrahlungsanlage.

Ausführungsformen der Erfindung mit Weiterbildungen gemäß den Merkmalen der abhängigen Ansprüche werden anhand der folgenden Zeichnung näher erläutert ohne jedoch darauf beschränkt zu sein. Es zeigen:
Fig. 1 eine schematische Übersicht über eine Bestrahlungsanlage, mit der ein Rescanning-Verfahren durchgeführt werden kann,
Fig. 2 ein Diagramm zur Darstellung einer inhomogene Dosisverteilung, die in einer der Isoenergieschichten zu applizieren ist,
Fig. 3 eine schematische Darstellung einer zu applizierenden Dosisverteilung in einem Zielbereich,
Fig. 4 bis Fig. 7 die Darstellung vier nacheinander auszuführender Rescan-Durchgänge mit einer Darstellung der Zielpunkte, die im jeweiligen Rescan-Durchgang angefahren werden,
Fig. 8 eine schematische Darstellung der Verfahrensschritte, die bei einer Ausführungsform der Erfindung durchgeführt werden.

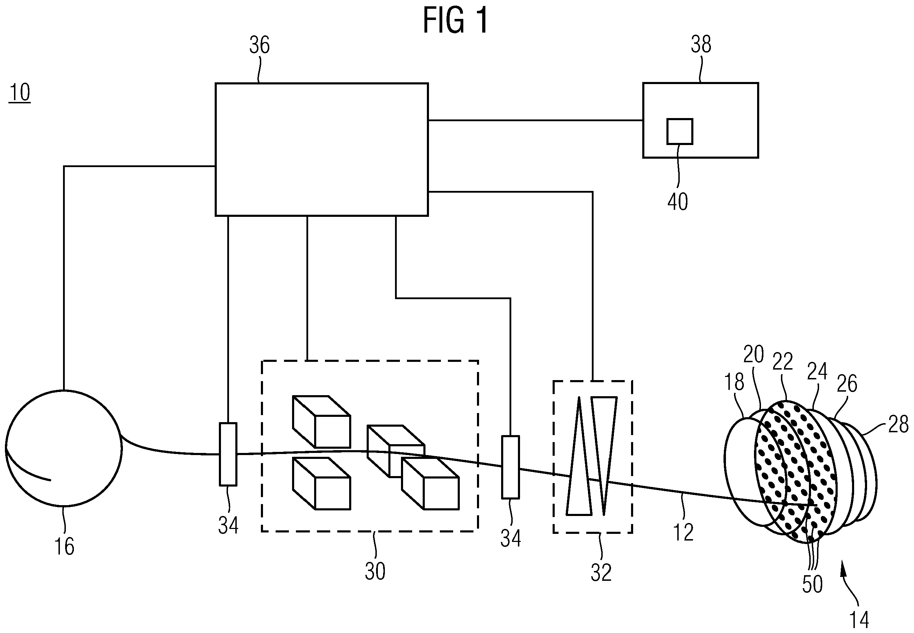

Fig. 1 zeigt in stark schematisierter Darstellung einen Aufbau einer Partikeltherapieanlage 10. Die Partikeltherapieanlage 10 wird zur Bestrahlung eines auf einer Positioniervorrichtung angeordneten Körpers mit einem Strahl aus Partikeln eingesetzt, der im Folgenden als Partikelstrahl 12 bezeichnet ist. Insbesondere kann als Zielvolumen 14 ein tumorerkranktes Gewebe eines Patienten mit dem Partikelstrahl 12 bestrahlt werden. Es ist ebenfalls vorgesehen, die Partikelstrahlanlage 10 zur Bestrahlung eines nicht-lebenden Körpers, insbesondere eines Wasserphantoms oder eines anderen Phantoms einzusetzen. Die Bestrahlung des Wasserphantoms kann beispielsweise zu Zwecken der Überprüfung und Verifizierung von Bestrahlungsparametern vor und/oder nach einer erfolgten Bestrahlung eines Patienten erfolgen. Es ist aber auch vorgesehen, andere Körper, insbesondere Versuchsaufbauten wie beispielsweise Zellkulturen oder Bakterienkulturen zu Forschungszwecken mit dem Partikelstrahl 12 zu bestrahlen. In allen Fällen kann es sich um bewegte oder ruhende Körper handeln.

Die Partikeltherapieanlage 10 weist typischerweise eine Beschleunigereinheit 16 auf, z.B. ein Synchrotron, ein Zyklotron oder einen sonstigen Beschleuniger, der einen Partikelstrahl 12 mit der zur Bestrahlung notwendigen Energie bereitstellt. Als Partikel werden vornehmlich Teilchen wie beispielsweise Protonen, Pionen, Heliumionen, Kohlenstoffionen oder Ionen anderer Elemente eingesetzt. Typischerweise hat ein Partikelstrahl 12 einen Strahldurchmesser von 3-10 mm.

In dem zu bestrahlenden Zielvolumen 14 sind Schichten 18, 20, 22, 24, 26 und 28 angedeutet, welche Isoenergieschichten entsprechen. Eine Isoenergieschicht 18, 20, 22, 24, 26 oder 28 ist durch die Eindringtiefe des Partikelstrahls 12 bei einer bestimmten Energie des Partikelstrahls 12 gekennzeichnet. Jede Isoenergieschicht 18, 20, 22, 24, 26, 28 stellt bei dem hier gezeigten Beispiel einen Zielbereich innerhalb des Zielvolumens 14 dar, der im Rescanning-Verfahren bestrahlt werden soll.

Als Scanverfahren wird bevorzugt ein Rasterscan-Verfahren verwendet, bei dem ein Partikelstrahl 12 von Zielpunkt 50 zu Zielpunkt 50 geführt wird ohne zwangsläufige Abschaltung bei einem Übergang von einem Zielpunkt 50 zum nächsten. Es können auch Spotscan-Verfahren mit Abschaltung des Partikelstrahls zwischen den einzelnen Zielpunkten 50 oder andere ScanVerfahren wie beispielsweise kontinuierliche Scanverfahren eingesetzt werden, um den Zielbereich mit einem Rescanning-Verfahren zu bestrahlen. In Fig. 1 sind einige Zielpunkte 50 der mittleren Isoenergieschicht 22 im Zielvolumen 14 dargestellt, welche sukzessive mit dem Partikelstrahl 12 angefahren werden.

Der Partikelstrahl 12 wird in seiner lateralen Auslenkung mithilfe von Scan-Magneten 30 beeinflusst, d.h. in seiner Position senkrecht zu Strahlverlaufsrichtung, auch als x- und y-Richtung bezeichnet, abgelenkt. Weiterhin kann eine Energiemodulationsvorrichtung 32 vorgesehen sein, mit der die Energie des Partikelstrahls 12 schnell geändert werden kann, so dass die Eindringtiefe des Partikelstrahls 12 variiert werden kann. Auf diese Weise ist auch ein Rescanning in Strahlrichtung des Partikelstrahls 12 möglich ("volumetrisches Rescanning", d.h. der Strahlpfad muss nicht innerhalb einer Isoenergieschicht verlaufen).

Die Bestrahlungsanlage 10 weist ferner eine Ablaufsteuerung 36 und Detektoren 34 zur Überwachung der Strahlparameter auf. Die Anordnung der hier gezeigten Komponenten der Partikelstrahlanlage 10 ist lediglich beispielhaft. Auch Aufbauten einer anderen Anordnung sind denkbar.

Die Ablaufsteuerung 36, also das Kontrollsystem der Anlage, steuert die einzelnen Komponenten der Anlage, wie beispielsweise den Beschleuniger 16, die Scan-Magnete 30 und sammelt Messdaten wie beispielsweise die Daten der Detektoren 34 zur Überwachung der Strahlparameter. Üblicherweise erfolgt die Steuerung basierend auf einem Bestrahlungsplan 40, der mithilfe einer Bestrahlungsplanungseinrichtung 38 ermittelt und bereitgestellt wird.

In einer hier gezeigten Partikeltherapieanlage 10 können Ausführungsformen der Erfindung implementiert werden. Mögliche Ausführungsformen werden anhand der nachfolgenden Zeichnung näher erläutert.

Das Zielvolumen 14 in Fig. 1 weist eine ellipsoide Form auf. Wenn das Zielvolumen 14 insgesamt mit einer homogenen Solldosis bestrahlt werden soll, bedeutet dies für die mittlere Isoenergieschicht 22, dass die in der mittleren Isoenergieschicht 22 zu applizierende Dosisverteilung inhomogen ist. Dies rührt daher, dass der zentrale Bereich der mittleren Isoenergieschicht 22 bereits durch eine geringe Dosis belastet wird, wenn die in Strahlrichtung hinter der mittleren Isoenergieschicht 22 liegenden Isoenergieschichten 24, 26, 28 bestrahlt werden. Für die mittlere Isoenergieschicht 22 ist die zu applizierende Dosis daher am Rand größer als im Zentrum.

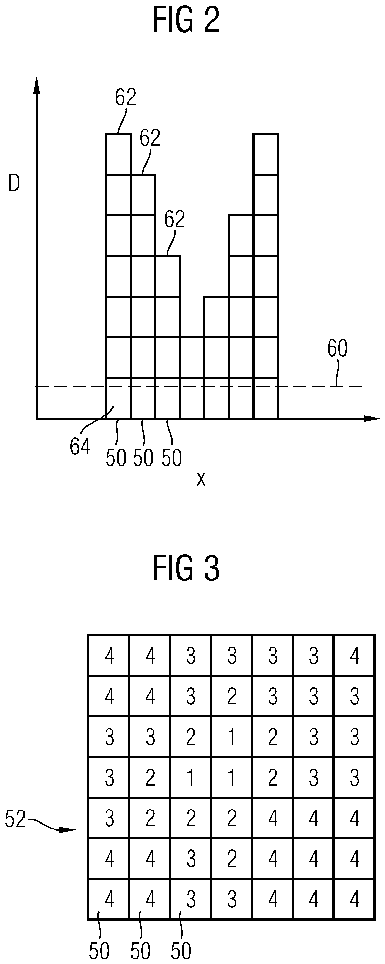

Die Dosisverteilung, die bei der mittleren Isoenergieschicht 22 zu applizieren ist, ist für einige Zielpunkte der mittleren Isoenergieschicht in Fig. 2 dargestellt. Die x-Achse kennzeichnet dabei den Ort x der Zielpunkte entlang einer Linie innerhalb der mittleren Isoenergieschicht, die y-Achse die zu applizierende Gesamtdosis D.

Gestrichelt eingezeichnet ist ein Schwellwert 60, der die minimale Fluenz angibt - und hierüber auch die minimale zu applizierende Dosis festlegt -, deren Applikation durch die Messinstrumente der Partikeltherapieanlage 10 mit der gebotenen Genauigkeit überwacht werden kann.

Die zu applizierende, inhomogene Gesamtdosis 62 für die mittlere Isoenergieschicht 22 ist dabei derart gewählt, dass die in den Zielpunkten 50 jeweils zu applizierende Gesamtdosis 62 stets ein ganzzahliges Vielfaches einer Einzeldosis 64 ist. Diese Wahl wird im Allgemeinen während der Bestrahlungsplanung getroffen und ist deshalb im Allgemeinen problemlos möglich, da die Einzeldosis 64 so klein gewählt ist, dass sie knapp oberhalb des Schwellwertes 60 liegt, also üblicherweise deutlich kleiner als die zu applizierende Gesamtdosis 62 ist. Die Einzeldosis 64 kann z.B. unterhalb des 1,5 fachen des Schwellwertes 60 liegen. Die Wahl der Einzeldosis 64 bewirkt, dass einerseits sichergestellt ist, dass die Dosisapplikation mit gebotener Genauigkeit überwacht werden kann, und dass andererseits möglichst viele Rescan-Durchgänge gefahren werden können, um die Gesamtdosis 62 für die mittlere Isoenergieschicht 22 zu applizieren.

Die Isoenergieschicht wird im Rescanning-Verfahren bestrahlt. Ein Zielpunkt 50 wird dabei während der verschiedenen Rescan-Durchgänge insgesamt sooft angefahren, bis die Gesamtdosis 62 für diesen Zielpunkt 50 erreicht worden ist. Bei jedem Anfahren eines Zielpunktes 50 kann die gesamte Einzeldosis 64 aufgrund des ganzzahligen Verhältnisses appliziert werden.

Die Vorgabe der Einzeldosis bewirkt, dass proximale Schichten, welche im allgemeinen mit einer geringeren Dosis bestrahlt werden, da diese Schichten mit einer Vor-Dosis belegt sind, mit weniger Rescan-Durchgängen bestrahlt werden. Dies ist allerdings vertretbar, da diese Bereiche bereits durch die Bestrahlung distaler Schichten bereits indirekt in vielfachen Rescan-Durchgängen bestrahlt worden sind. Des Weiteren werden in proximalen Schichten in der Regel Einstellungen verwendet, die eine genauere Dosismessung und demnach kleinere Schwellwerte 60 erlauben. Dies kann z.B. über eine geringere Intensität des Partikelstrahls und durch die Wahl eines sensitiveren Messbereichs der Messvorrichtungen wie Ionisationskammern erreicht werden, mit welchen die Dosisapplikation des Partikelstrahls überwacht wird.

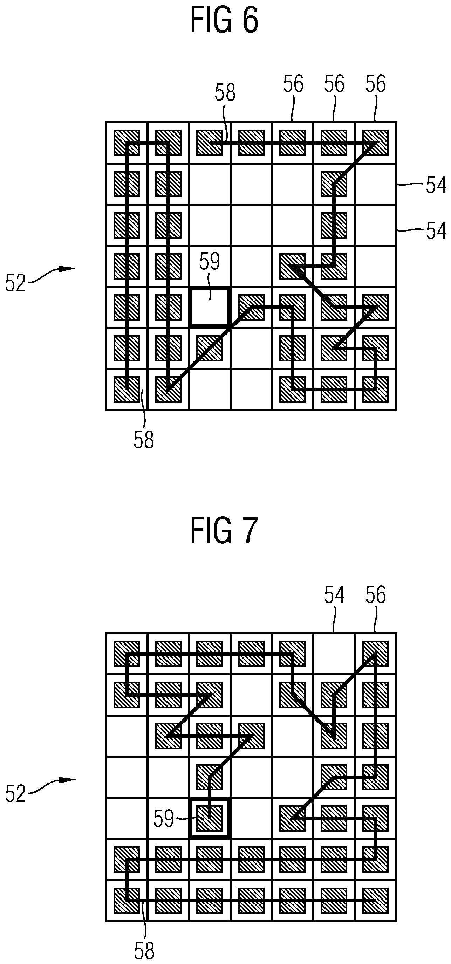

Mithilfe von Fig. 3 bis 8 wird eine Ausführungsform des erfindungsgemäßen Verfahrens erläutert anhand der Darstellung einer fiktiven, quadratischen Isoenergieschicht 52. Die dargestellte Isoenergieschicht 52 ist eine starke Vereinfachung einer üblicherweise in Realität auftretenden Isoenergieschicht. Das Grundprinzip kann jedoch besser erläutert werden.

Fig. 3 zeigt für jeden Zielpunkt 50 die zu applizierende Gesamtdosis als Vielfaches einer Einzeldosis. Zu erkennen ist, dass die Gesamtdosis in den Randbereichen der quadratischen Isoenergieschicht 52 größer ist (also bis zu einem 4-fachen der Einzeldosis) als im Zentrum (lediglich das 1-fache der Einzeldosis). Dies entspricht vom Grundprinzip in etwa der in Fig. 2 dargestellten inhomogenen Dosisverteilung.

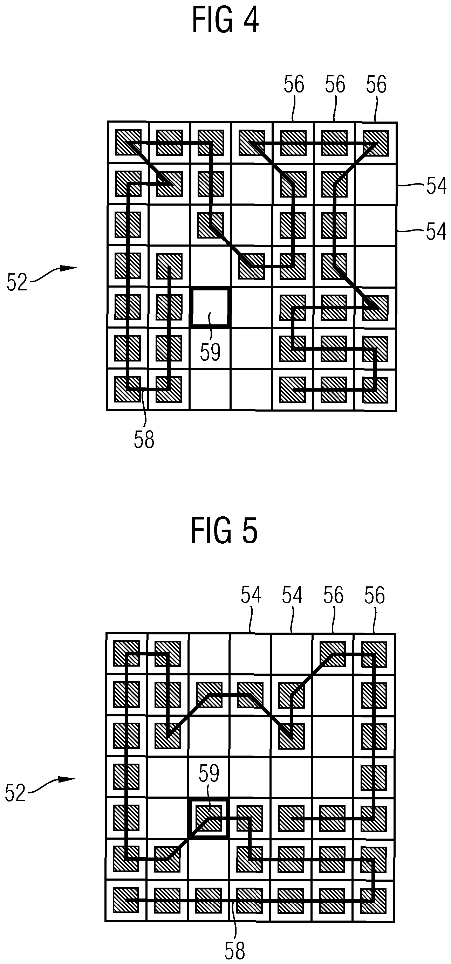

Fig. 4 bis Fig. 7 zeigt das Anfahren der Zielpunkte für vier nacheinander auszuführende Rescan-Durchgänge. Ein belegtes Kästchen 56 bedeutet dabei, dass der Zielpunkt bei dem entsprechenden Rescan-Durchgang angefahren und dass bei dem Zielpunkt die Einzeldosis appliziert wird, ein nicht-belegtes Kästchen 54 bedeutet hingegen, dass der Zielpunkt bei dem Rescan-Durchgang nicht angefahren, d.h. ausgelassen, wird.

Die Zielpunkte 50 wurden dabei derart auf die Rescan-Durchgänge verteilt, dass bei jedem Rescan-Durchgang eine möglichst gleichbleibende Anzahl an Zielpunkten 50 angefahren wird. Weiterhin wurde bei der Verteilung des Anfahrens der Zielpunkte 50 auf die Rescan-Durchgänge darauf geachtet, dass sich immer ein durchgehender Scan-Pfad 58 finden lässt, sodass eine Bestrahlung in einem Rescan-Durchgang ohne Unterbrechung möglich ist.

Durch die gleichmäßige Verteilung ergibt sich insbesondere bei bestimmten Zielpunkten, dass es Rescan-Durchgänge gibt, in denen dieser Zielpunkt nicht angefahren wird und welche vor einem Rescan-Durchgang liegen, bei welchem dieser Zielpunkt angefahren wird. Dies ist beispielsweise bei einem der zentralen Zielpunkte 59 der Fall. Dieser Zielpunkt 59 wird nur beim zweiten und beim vierten Rescan-Durchgang angefahren, nicht aber beim ersten und beim dritten Rescan-Durchgang.

Insbesondere bewirkt die Aufteilung, dass einige Zielpunkte erst in späteren Rescan-Durchgängen angefahren werden. Dies ermöglicht es, günstige Scan-Pfade 58 zu finden. Als Methoden hierfür können bekannte Algorithmen, wie sie auch bei allgemeinen Traveling-Salesman Problemen eingesetzt werden, verwendet werden. Vor allem bei komplexen Zielbereichen, wie sie in Realität auftreten, kann sonst der Fall eintreten, dass in einem Rescan-Durchgang nicht-zusammenhängende Inseln aus Zielpunkten angefahren werden müssten. Dies kann durch die beschriebene Aufteilung des Anfahrens der Zielpunkte 50 auf verschiedene Rescan-Durchgänge verhindert werden.

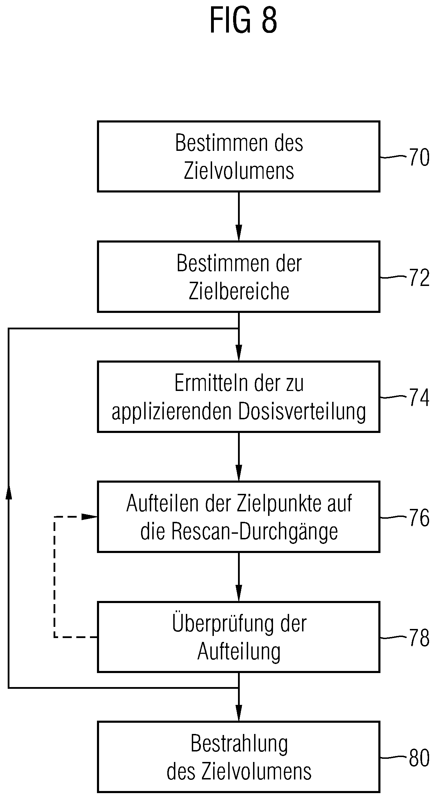

Fig. 8 zeigt eine schematische Darstellung der Verfahrensschritte, welche bei der Planung einer Bestrahlung im Rescanning-Verfahren durchgeführt werden.

Zuerst wird ein Zielvolumen in dem zu bestrahlenden Objekt definiert (Schritt 70). Anschließend wird das Zielvolumen in Zielbereiche unterteilt, welche jeweils in mehreren Rescan-Durchgängen bestrahlt werden sollen (Schritt 72).

Für jeden dieser Zielbereiche wird die zu applizierende Dosisverteilung ermittelt (Schritt 74). Dabei kann eine Einzeldosis bestimmt werden, welche die Dosis beschreibt, die in einem Zielpunkt pro Anfahren des Zielpunktes abgegeben wird. Diese Einzeldosis kann aber auch beispielsweise von vornherein festgelegt sein. Die Ermittlung der zu applizierenden Dosisverteilung kann auch unter bestimmten Randbedingungen erfolgen, beispielsweise kann festgelegt werden, dass pro Zielpunkt die zu applizierende Gesamtdosis ein ganzzahliges Vielfaches einer Einzeldosis ist. Aus der Einzeldosis und aus der Gesamtdosis pro Zielpunkt ergibt sich die Anzahl der Rescan-Durchgänge, die appliziert werden müssen, um im Zielbereich die gewünschte Dosisverteilung zu applizieren.

Anschließend werden die Zielpunkte bzw. das Anfahren der Zielpunkte auf die Rescan-Durchgänge aufgeteilt, und zwar derart, dass bei zumindest einem Zielpunkt, der nicht bei jedem Rescan-Durchgang angefahren wird, vor dem letzten Anfahren des Zielpunktes zumindest ein Rescan-Durchgang liegt, während dem dieser Zielpunkt nicht angefahren wird (Schritt 76).

Nachdem das Anfahren der Zielpunkte auf die Rescan-Durchgänge aufgeteilt wurde, wird geprüft, ob bei jedem Rescan-Durchgang die Zielpunkte mit einem Scan-Pfad angefahren werden können, welcher vordefinierte Kriterien erfüllt (Schritt 78). Beispielsweise kann geprüft werden, ob die Anzahl der Unterbrechungen des Strahls, die bei der Ausführung eines Scan-Pfades notwendig sind, unterhalb eines Wertes liegen oder minimiert sind.

Falls die vordefinierten Kriterien nicht erfüllt sind, kann die Aufteilung der Zielpunkte bzw. das Anfahren der Zielpunkte geändert werden, und zwar derart, dass die sich ergebenden Scan-Pfade die vordefinierten Kriterien besser erfüllen.

Die Aufteilung der Zielpunkte auf Rescan-Durchgänge wird für jeden Zielbereich in ähnlicher Weise wiederholt. Anschließend kann die Bestrahlung des Zielvolumens bzw. der Zielbereiche des Zielvolumens gemäß der Bestrahlungsplanung erfolgen (Schritt 80).

Das Verfahren lässt sich mit anderen Verfahren, die das Rescanning betreffen, kombinieren. Beispielsweise können die Rescan-Durchgänge zeitlich so gelegt werden, dass eine Desynchronisation mit der Bewegung des Zielvolumens auftritt, es kann mit einem Verfahren wie in der US 20080078942 A1 offenbart kombiniert werden, etc. Die Ausführungsbeispiele, die nicht unter den Schutzumfang der beigefügten Ansprüche fallen, dienen lediglich zu illustrativen Zwecken und sind nicht Gegenstand der gegenwärtigen Erfindung. Die Erfindung wird durch die folgenden Ansprüche definiert.

### Bezugszeichenliste

10 Partikeltherapieanlage
11 Partikelstrahl
14 Zielvolumen
16 Beschleunigereinheit
18, 20, 22, 24, 26, 28 Isoenergieschicht
30 Scan-Magnete
32 Energiemodulationsvorrichtung
34 Detektor
36 Ablaufsteuerung
38 Bestrahlungsplanungsvorrichtung
40 Bestrahlungsplan
50 Zielpunkte
52 quadratische Isoenergieschicht
54 leeres Kästchen
56 belegtes Kästchen
58 Scanpfad
59 zentraler Zielpunkt
60 Schwellwert
62 Gesamtdosis
64 Einzeldosis
70 Schritt 70
72 Schritt 72
74 Schritt 74
76 Schritt 76
78 Schritt 78
80 Schritt 80

## Patentansprüche

1. Verfahren zur Bestrahlungsplanung eines Zielvolumens (14) mittels einer Rechnereinheit umfassend folgende Schritte:
- Festlegen eines Zielbereichs (18, 20, 22, 24, 26, 28) mit einzeln anfahrbaren Zielpunkten (50),
- Festlegen einer Anzahl von Rescan-Durchgängen, in denen der Zielbereich (18, 20, 22, 24, 26, 28) mehrfach abgescannt wird, derart, dass die Zielpunkte (50) des Zielbereichs (18, 20, 22, 24, 26, 28) während der Rescan-Durchgänge unterschiedlich oft angefahren werden, wodurch zumindest ein Teil der Zielpunkte (54) nicht bei jedem Rescan-Durchgang angefahren wird,
wobei das Anfahren der Zielpunkte (50) derart auf die Rescan-Durchgänge aufgeteilt wird, dass bei zumindest einem Zielpunkt (59), der nicht in jedem Rescan-Durchgang angefahren wird, vor dem letzten Rescan-Durchgang, bei dem dieser Zielpunkt (59) angefahren wird, zumindest ein weiterer Rescan-Durchgang liegt, bei dem dieser Zielpunkt (59) nicht angefahren wird.

2. Verfahren nach Anspruch 1, wobei ein Zielpunkt (59) nicht bei dem ersten Rescan-Durchgang angefahren wird.

3. Verfahren nach Anspruch 1 oder 2, wobei bei einem Zielpunkt (59), der bei zumindest zwei Rescan-Durchgängen angefahren wird, zwischen diesen zwei Rescan-Durchgängen zumindest ein weiterer Rescan-Durchgang liegt, bei dem der Zielpunkt (59) nicht angefahren wird.

4. Verfahren nach einem der Ansprüche 1 bis 3, wobei bei jedem Rescan-Durchgang einige Zielpunkte nicht angefahren werden.

5. Verfahren nach einem der Ansprüche 1 bis 4, wobei das Anfahren der Zielpunkte (50) derart auf die Rescan-Durchgänge aufgeteilt wird, dass ein Scan-Pfad (58), mit dem in einem der Rescan-Durchgänge die anzufahrenden Zielpunkte (56) angefahren werden, ein vordefiniertes Kriterium erfüllt.

6. Verfahren nach Anspruch 5, wobei das vordefinierte Kriterium umfasst, dass in einem Scan-Pfad (58) ein Abstand zwischen zwei nacheinander anzufahrenden Zielpunkten unterhalb eines Schwellwertes liegt.

7. Verfahren nach einem der Ansprüche 1 bis 6, wobei bei einem Zielpunkt (50) eine zu applizierende Gesamtdosis (62) ein ganzzahliges Vielfaches der bei diesem Zielpunkt (50) zu applizierenden Einzeldosis (64) ist.

8. Verfahren nach einem der Ansprüche 1 bis 7, wobei bei den Zielpunkten (50) des Zielbereichs bei jedem Anfahren jeweils eine gleich große Einzeldosis (64) appliziert wird.

9. Verfahren nach Anspruch 7 oder 8, wobei die Einzeldosis als Vielfaches eines von einer Messvorrichtung für die Überwachung einer Partikelstrahleigenschaft vorgegebenen Schwellwertes gewählt wird.

10. Verfahren zur Bestrahlung eines Zielbereichs (18, 20, 22, 24, 26, 28) mittels einer Steuerungsvorrichtung mit Steuerungsrechner in einem nicht-lebenden Zielvolumen (14),
bei welchem der Zielbereich (18, 20, 22, 24, 26, 28) einzeln anzufahrende Zielpunkte (50) umfasst,
wobei der Zielbereich (18, 20, 22, 24, 26, 28) mit einer Vielzahl von Rescan-Durchgängen bestrahlt wird, durch welche der Zielbereich (18, 20, 22, 24, 26, 28) mehrfach abgescannt wird,
wobei die Zielpunkte (50) des Zielbereichs (18, 20, 22, 24, 26, 28) während der Rescan-Durchgänge unterschiedlich oft angefahren werden, wodurch zumindest ein Teil der Zielpunkte (54) nicht bei jedem Rescan-Durchgang angefahren wird, und
wobei bei zumindest einem Zielpunkt (59), der nicht in jedem Rescan-Durchgang angefahren wird, vor dem letzten Rescan-Durchgang, bei dem dieser Zielpunkt (59) angefahren wird, zumindest ein weiterer Rescan-Durchgang ausgeführt wird, bei dem dieser Zielpunkt (59) nicht angefahren wird.

11. Verfahren nach Anspruch 10, wobei der zumindest ein Zielpunkt (59) nicht bei dem ersten Rescan-Durchgang angefahren wird oder wobei der zumindest eine Zielpunkt (59) bei mindestens zwei Rescan-Durchgängen angefahren wird, derart, dass zwischen diesen zwei Rescan-Durchgängen zumindest ein weiterer Rescan-Durchgang liegt, bei dem der Zielpunkt (59) nicht angefahren wird.

12. Verfahren nach Anspruch 10 oder 11,
wobei die bei einem Zielpunkt (50) die zu applizierende Gesamtdosis (62) als ein Vielfaches der bei diesem Zielpunkt (50) zu applizierenden Einzeldosis (64) appliziert wird.

13. Bestrahlungsplanungsvorrichtung (38) mit einer Rechnereinheit, welche zur Durchführung eines Verfahrens nach einem der Ansprüche 1 bis 9 ausgebildet ist.

14. Steuerungsvorrichtung (36) für eine Bestrahlungsanlage (10), mit einem Steuerungsrechner, der die Bestrahlungsanlage (10) bei einer Bestrahlung derart steuert, dass ein Verfahren nach einem der Ansprüche 10 bis 12ausgeführt wird.

15. Bestrahlungsanlage (10) mit einer Steuerungsvorrichtung (36) nach Anspruch 14.

## Claims

1. A method for irradiation planning for a target volume (14) using a computer unit, comprising the steps of:
- defining a target region (18, 20, 22, 24, 26, 28) with target points (50) that can be targeted individually;
- defining a number of rescanning passes in which the target region (18, 20, 22, 24, 26, 28) is scanned multiple times, so that the target points (50) of the target region (18, 20, 22, 24, 26, 28) are targeted a different number of times during the rescanning passes, whereby at least part of the target points (54) is not targeted in each rescanning pass; wherein the targeting of the target points (50) is distributed among the rescanning passes such that for at least one target point (59) that is not targeted in each rescanning pass, there is, prior to the final rescanning pass in which this target point (59) is targeted, at least one further rescanning pass in which this target point (59) is not targeted.

2. The method according to claim 1, wherein one target point (59) is not targeted in the first rescanning pass.

3. The method according to claim 1 or 2, wherein for one target point (59) that is targeted in at least two rescanning passes, there is, between these two rescanning passes, at least one further rescanning pass in which the target point (59) is not targeted.

4. The method according to any one of claims 1 to 3, wherein in each rescanning pass some target points are not targeted.

5. The method according to any one of claims 1 to 4, wherein the targeting of the target points (50) is distributed among the rescanning passes such that a scanning path (58) along which the target points (56) to be targeted in one of the rescanning passes are targeted, meets a predetermined criterion.

6. The method according to claim 5, wherein the predetermined criterion is that along a scanning path (58) a spacing between two target points that are to be targeted successively is below a threshold value.

7. The method according to any one of claims 1 to 6, wherein a total dose to be applied (62) at a target point (50) is an integral multiple of the single dose (64) to be applied at this target point (50).

8. The method according to any one of claims 1 to 7, wherein upon each targeting the same respective single dose is applied at the target points (50) of the target region.

9. The method according to claim 7 or 8, wherein the single dose is selected as a multiple of a threshold value that is predefined by a measuring device for monitoring a particle beam property.

10. A method for irradiating a target region (18, 20, 22, 24, 26, 28) using a control device including a control computer, in a non-living target volume (14),
wherein the target region (18, 20, 22, 24, 26, 28) comprises target points (50) to be targeted individually;
wherein the target region (18,20,22,24,26,28)
is irradiated by a plurality of rescan passes whereby the target region (18, 20, 22, 24, 26, 28) is scanned multiple times;
wherein the target points (50) of the target region (18, 20, 22, 24, 26, 28) are targeted a different number of times during the rescanning passes, whereby at least part of the target points (54) is not targeted in each rescanning pass; and
wherein for at least one target point (59) that is not targeted in each rescanning pass, there is, prior to the final rescanning pass in which this target point (59) is targeted, at least one further rescanning pass in which this target point (59) is not targeted.

11. The method according to claim 10, wherein said at least one target point (59) is not targeted in the first rescanning pass, or wherein the at least one target point (59) is targeted in at least two rescanning passes in a manner so that between these two rescanning passes there is at least one further rescanning pass in which the target point (59) is not targeted.

12. The method according to claim 10 or 11,
wherein the total dose to be applied (62) at a target point (50) is a multiple of the single dose (64) to be applied at this target point (50).

13. An irradiation planning device (38), comprising a computer unit which is configured for performing a method according to any one of claims 1 to 9.

14. A control device (36) for an irradiation system (10), comprising a control computer which controls the irradiation system (10) during an irradiation so as to perform a method according to anyone of claims 10 to 12.

15. An irradiation system (10), comprising a control device (36) according to claim 14.

## Revendications

1. Procédé de planification d'irradiation d'un volume cible (14) au moyen d'une unité d'ordinateur, comprenant les étapes suivantes :
- définition d'une zone cible (18, 20, 22, 24, 26, 28) avec des points cibles (50) pouvant être visés individuellement,
- définition d'un nombre de passages de balayage répété, lors desquels la zone cible (18, 20, 22, 24, 26, 28) est balayée à plusieurs reprises, de telle sorte que les points cibles (50) de la zone cible (18, 20, 22, 24, 26, 28) soient visés avec des fréquences différentes au cours des passages de balayage répété, ce qui a pour effet qu'au moins une partie des points cibles (50) n'est pas visée lors de chaque passage de balayage répété,
selon lequel le fait de viser des points cibles (50) est réparti sur les passages de balayage répété (59) de manière telle que pour au moins un point cible (59) qui n'est pas visé lors de chaque passage de balayage répété, il y ait, avant le dernier passage de balayage répété lors duquel ce point cible (59) est visé, au moins un autre passage de balayage répété lors duquel ce point cible (59) n'est pas visé.

2. Procédé selon la revendication 1, selon lequel un point cible (59) n'est pas visé lors du premier passage de balayage répété.

3. Procédé selon la revendication 1 ou 2, selon lequel, pour un point cible (59) qui est visé au moins lors de deux passages de balayage répété, au moins un autre passage de balayage répété est intercalé entre ces deux passages de balayage répété, lors duquel le point cible (59) n'est pas visé.

4. Procédé selon l'une des revendications 1 à 3, selon lequel, lors de chaque passage de balayage répété, quelques points cibles ne sont pas visés.

5. Procédé selon l'une des revendications 1 à 4, selon lequel le fait de viser des points cibles (50) est réparti sur les passages de balayage répété, de manière telle qu'un chemin de balayage (58), par lequel les points cibles (56) à viser sont visés lors d'un des passages de balayage répété, répond à un critère prédéfini.

6. Procédé selon la revendication 5, selon lequel le critère prédéfini inclut le fait que dans un chemin de balayage (58), une distance entre deux points cibles devant être visés l'un après l'autre est inférieure à une valeur seuil.

7. Procédé selon l'une des revendications 1 à 6, selon lequel, pour un point cible (50), une dose totale (62) à appliquer représente un multiple entier de la dose individuelle (64) à appliquer à ce point cible (50).

8. Procédé selon l'une des revendications 1 à 7, selon lequel, pour les points cibles (50) de la zone cible, une même dose individuelle (64) est appliquée lors de chaque visée.

9. Procédé selon la revendication 7 ou 8, selon lequel la dose individuelle est choisie comme un multiple d'une valeur seuil prédéterminée par un dispositif de mesure pour la surveillance d'une propriété de faisceau de particules.

10. Procédé d'irradiation d'une zone cible (18, 20, 22, 24, 26, 28) au moyen d'un dispositif de commande avec un ordinateur de commande, dans un volume cible (14) non vivant,
selon lequel la zone cible (18, 20, 22, 24, 26, 28) comporte des points cibles (50) à viser individuellement,
selon lequel la zone cible (18, 20, 22, 24, 26, 28) est irradiée avec un grand nombre de passages de balayage répété, par lesquels la zone cible (18, 20, 22, 24, 26, 28) est balayée à plusieurs reprises,
selon lequel les points cibles (50) de la zone cible (18, 20, 22, 24, 26, 28) sont visés avec des fréquences différentes au cours des passages de balayage répété, ce qui a pour effet qu'au moins une partie des points cibles (54) n'est pas visée lors de chaque passage de balayage répété, et
selon lequel, pour au moins un point cible (59) qui n'est pas visé lors de chaque passage de balayage répété, il y ait, avant le dernier passage de balayage répété lors duquel ce point cible (59) est visé, au moins un autre passage de balayage répété lors duquel ce point cible (59) n'est pas visé.

11. Procédé selon la revendication 10, selon lequel le point cible (59), au nombre d'au moins un, n'est pas visé lors du premier passage de balayage répété, ou selon lequel le point cible (59), au nombre d'au moins un, est visé au moins lors de deux passages de balayage répété, de manière telle qu'entre ces deux passages de balayage répété, il y ait au moins un autre passage de balayage répété lors duquel le point cible (59) n'est pas visé.

12. Procédé selon la revendication 10 ou 11, selon lequel la dose totale (62) à appliquer à un point cible (50) est appliquée en tant que multiple de la dose individuelle (64) à appliquer à ce point cible (50).

13. Dispositif de planification d'irradiation (38) comprenant une unité d'ordinateur qui est agencée pour la mise en oeuvre d'un procédé selon l'une des revendications 1 à 9.

14. Dispositif de commande (36) pour une installation de planification d'irradiation (10), comprenant un ordinateur de commande qui, lors d'une irradiation, commande l'installation d'irradiation (10) de manière à mettre en oeuvre un procédé selon l'une des revendications 10 à 12.

15. Installation d'irradiation (10) comprenant un dispositif de commande (36) selon la revendication 14.
